# EUROPÄISCHE PATENTSCHRIFT

(11) **EP 4 181 836 B1**
(45) Veröffentlichungstag und Bekanntmachung des Hinweises auf die Patenterteilung: **02.07.2025**
(21) Anmeldenummer: 21754712.4
(22) Anmeldetag: 20.07.2021
(51) Int. Cl.: A61F 2/60, A61F 2/64, A61F 2/68, A61F 2/50, A61F 2/76

(54) **VERFAHREN ZUR STEUERUNG EINER PROTHESE ODER ORTHESE**
METHOD FOR CONTROLLING A PROSTHESIS OR ORTHESIS
PROCÉDÉ DE COMMANDE D'UNE PROTHÈSE OU ORTHÈSE

(30) Priorität: 20.07.2020 DE 102020004339
(43) Veröffentlichungstag der Anmeldung: 24.05.2023
(73) Patentinhaber: Otto Bock Healthcare Products GmbH, 1110 Wien (AT)
(72) Erfinder: HOFMANN, Thomas, 1030 Wien (AT); SEIFERT, Dirk, 1070 Wien (AT); ZARLING, Sven, 37115 Duderstadt (DE)
(74) Vertreter: Gramm, Lins & Partner Patent- und Rechtsanwälte PartGmbB
(86) Internationale Anmeldenummer: PCT/EP2021/070283
(87) Internationale Veröffentlichungsnummer: WO 2022/018091

(56) Entgegenhaltungen:
- CA-A1- 2 772 620
- US-B2- 8 852 292
- US-B2- 9 066 819
- US-B2- 9 737 419

## Beschreibung

Die Erfindung betrifft ein Verfahren zum Steuern einer Prothese oder Orthese einer unteren Extremität mit einem Oberteil und einem mit dem Oberteil über ein Kniegelenk verbundenes Unterteil, das um eine Gelenkachse relativ zu dem Oberteil verschwenkbar gelagert ist, wobei zwischen dem Oberteil und dem Unterteil eine verstellbare Widerstandseinrichtung angeordnet ist, über die beim Gehen auf der Grundlage von Sensordaten ein Flexionswiderstand in einer frühen und mittleren Standphase nach einem initialen Fersenkontakt bis zur mittleren Standphase verändert wird.

Künstliche Kniegelenke werden in Prothesen und Orthesen sowie in Exoskeletten als Sonderfall von Orthesen verwendet. Ein künstliches Kniegelenk weist ein Oberteil und ein Unterteil auf, die relativ zueinander verschwenkbar um eine Gelenkachse, die Knieachse, gelagert sind. Im einfachsten Fall ist das Kniegelenk als ein Einachskniegelenk ausgebildet, bei dem beispielsweise ein Bolzen oder zwei auf einer Schwenkachse angeordnete Lagerstellen eine einzelne Knieachse bilden. Ebenfalls sind künstliche Kniegelenke bekannt, die keine festgelegte Drehachse zwischen Oberteil und Unterteil ausbilden, sondern entweder abgleitende oder abrollende Oberflächen oder eine Vielzahl von gelenkig miteinander verbundenen Lenkern aufweisen. Um die Bewegungseigenschaften der Kniegelenke beeinflussen zu können und ein an das natürliche Gangverhalten angenähertes Bewegungsverhalten der Orthese oder Prothese bzw. dem Exoskelett zu erhalten, sind zwischen dem Oberteil und dem Unterteil Widerstandseinrichtungen vorgesehen, über die der jeweilige Widerstand verändert werden kann. Rein passive Widerstandseinrichtungen sind passive Dämpfer, beispielsweise Hydraulikdämpfer, Pneumatikdämpfer oder Dämpfer, die auf Grundlage magnetorheologischer Effekte den Bewegungswiderstand verändern. Ebenfalls existieren aktive Widerstandseinrichtungen, beispielsweise Motoren oder andere Antriebe, die über eine entsprechende Verschaltung als Generator oder Energiespeicher betreibbar sind.

Die jeweiligen Kniegelenke, also die Prothesengelenke oder Orthesenkniegelenke, werden mit jeweiligen Anschlussmitteln an den Patienten festgelegt. Bei Prothesenkniegelenken erfolgt die Festlegung in der Regel über einen Oberschenkelschaft, der einen Gliedmaßenstumpf aufnimmt. Alternative Festlegungsarten sind ebenfalls möglich, beispielsweise durch osseointegrierte Anschlussmittel oder über Gurte und andere Einrichtungen. Bei Orthesen und Exoskeletten werden das Oberteil und Unterteil unmittelbar an dem Oberschenkel und dem Unterschenkel festgelegt. Die dafür vorgesehenen Befestigungseinrichtungen sind beispielsweise Gurte, Manschetten, Schalen oder Rahmenkonstruktionen. Orthesen können auch Fußteile zum Aufsetzen von eines Fußes oder Schuhes aufweisen. Die Fußteile können gelenkig an dem Unterteil gelagert sein.

Die DE 10 2013 011 080 A1 betrifft ein Verfahren zur Steuerung einer orthopädietechnischen Gelenkeinrichtung einer unteren Extremität mit einem Oberteil und einem gelenkig daran gelagerten Unterteil, zwischen denen eine Umwandlungseinrichtung angeordnet ist, über die während einer Verschwenkung des Oberteils relativ zu dem Unterteil mechanische Arbeit aus der Relativbewegung umgewandelt und zumindest in einem Energiespeicher gespeichert wird. Die gespeicherte Energie wird der Gelenkeinrichtung zeitversetzt wieder zugeführt, um im Verlauf der Bewegung die Verschwenkung von Oberteil und Unterteil zu unterstützen. Die Unterstützung der Relativbewegung erfolgt kontrolliert. Zusätzlich zu der Umwandlungseinrichtung kann ein separater Dämpfer in Gestalt eines Hydraulikdämpfers oder Pneumatikdämpfers vorgesehen sein, der verstellbar ausgebildet ist, sodass über die Dämpfereinrichtung der Widerstand während des Gehens sowohl in Flexionsrichtung als auch in Extensionsrichtung beeinflusst werden kann.

Ein künstliches Kniegelenk weist in der konstruktiv maximal erreichbaren Streckung einen Kniewinkel von 180° auf, eine Hyperextension, also ein Winkel auf der posterioren Seite von mehr als 180° ist in der Regel nicht vorgesehen. Das Verschwenken des Unterteils gegenüber dem Oberteil nach posterior wird als Knie-Flexion bezeichnet, ein Verschwenken nach anterior oder nach vorn in die Richtung als Extension. Bei einem Initialkontakt setzt der Fuß am Ende der Schwungphase zu Beginn der Standphase auf den Boden auf. Beim Gehen in der Ebene kommt es dabei zumeist zu einem sogenannten Fersenstoß, bei der der Fuß zuerst mit der Ferse aufsetzt.

Bleibt das künstliche Kniegelenk in einer gestreckten, geraden Stellung beim Fersenstoß, führt dies zu einer unmittelbaren Kraftdurchleitung in das Becken, was einerseits unangenehm ist und andererseits dem natürlichen Gangmuster widerspricht. Daher wird analog zum üblichen Gehen in der Ebene bei Prothesen und Orthesen eine sogenannte Standphasenflexion zugelassen, bei der nach dem Fersenstoß das Kniegelenk um die Gelenkachse einbeugt, gegebenenfalls entgegen einer Widerstandskraft über die Widerstandseinrichtung.

Aus der WO 2015/0101417 A1 ist ein Prothesenkniegelenk mit einem Oberteil und einem Unterteil bekannt, die über ein viergliedriges Gelenksystem verschwenkbar aneinander gelagert sind. Das Gelenksystem ist aus einer Ausgangsstellung entgegen einer Federkraft während einer Standphasenflexion verschwenkbar an dem Unterteil gelagert, wobei die Wirklinie der Federkraft so ausgerichtet ist, dass ein gegen eine Einbeugung des Gelenksystems wirkendes Moment vorliegt.

Die US 8 852 292 B2 betrifft ein orthopädietechnisches System mit einem Unterschenkelelement, einem Aktuator und einem Fußelement. Der Aktuator ist eingerichtet, die natürlichen Bewegungen eines gesunden Fußgelenks nachzuahmen. Eine Steuereinheit empfängt Sensordaten von einer Sensoreinheit. Die Steuereinheit detektiert Veränderungen des Untergrundes und gibt ein Ausgangssignal abhängig von den aktuellen Untergrundverhältnissen ab.

Die US 9 737 419 B2 betrifft ein Verfahren zur Steuerung einer Prothese oder Orthese mit einem Betätigungselement, das einen Motor umfasst, der in Reihe mit einem elastischen Element gekoppelt ist, um ein Drehmoment auf ein Gelenk aufzubringen. Mithilfe einer Steuereinheit wird ein Drehmomentfehler als Differenz zwischen einem Soll-Drehmoment und einem gemessenen Drehmoment bestimmt. Zur Bestimmung des Drehmomentfehlers wird ein Modell verwendet, in das eine elektromotorische Gegenkraft des Motors einfließt. Die an dem Motor angelegte Spannung wird durch die Steuereinheit gesteuert, um den Drehmomentfehler zu reduzieren.

Die CA 2 772 620 A1 betrifft eine aktive Prothese oder Orthese der unteren Extremität mit einem Oberschenkelelement und einem Unterschenkelelement, das über ein Kniegelenk mit dem Oberschenkelelement verbunden ist. Mithilfe eines Sensors und einer Steuereinheit wird die Position des Kniegelenks relativ zu einem Knöchelgelenk bestimmt. Wenn sich das Kniegelenk relativ zum Knöchelgelenk nach vorne bewegt, gibt die Steuereinheit ein Signal an einen Drehmotor, sodass an dem Kniegelenk ein Drehmoment appliziert wird, um den sitzenden Benutzer der Prothese oder Orthese beim Aufstehen zu unterstützen.

Die US 9 066 819 B2 betrifft eine Prothese der unteren Extremität mit einem das Bein umschließenden Schaft und einem über ein Kniegelenk schwenkbar dazu angeordneten Verbindungselement. An dem posterioren Ende des Verbindungselements ist ein Knöchelgelenk angeordnet, über das das Verbindungselement schwenkbar zu einer Fußprothese angeordnet ist. Die Prothese weist mehrere Sensoren und eine Steuereinheit auf, die eingerichtet ist, den Dämpfungswiderstand des Knöchelgelenks in Abhängigkeit von erfassten Sensordaten zu verändern.

Problematisch ist bei den Verfahren zur Steuerung bislang, dass der Flexionswiderstand in der Standphase dauerhaft eingestellt ist, sodass es bei abweichenden Gangsituationen zu Schwierigkeiten kommen kann, ein angenehmes Gangverhalten bereitzustellen.

Aufgabe der vorliegenden Erfindung ist es daher, ein Verfahren zur Steuerung einer Prothese oder Orthese einer unteren Extremität bereitzustellen, mit dem auf einfache Art und Weise ein verbessertes Gangverhalten für Nutzer mit künstlichen Kniegelenkes erreicht werden kann.

Erfindungsgemäß wird diese Aufgabe durch ein Verfahren mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das Verfahren zur Steuerung einer Prothese oder Orthese der unteren Extremität mit einem Oberteil und einem mit dem Oberteil über ein Kniegelenk verbundenes Unterteil, das um eine Gelenkachse relativ zu dem Oberteil verschwenkbar gelagert ist, wobei zwischen dem Oberteil und dem Unterteil eine verstellbare Widerstandseinrichtung angeordnet ist, über die beim Gehen auf der Grundlage von Sensordaten ein Flexionswiderstand in einer frühen und mittleren Standphase nach einem initialen Fersenkontakt bis zu der mittleren Standphase verändert wird, sieht vor, dass nach dem initialen Fersenkontakt der Flexionswiderstand auf einen Wert erhöht wird, bei dem eine weitere Flexion gesperrt oder zumindest verlangsamt wird, wobei der zeitliche Verlauf der Flexionswiderstandserhöhung und/oder der maximal erreichbare Flexionswinkel in Abhängigkeit von der Neigung des Untergrundes oder einer zu überwindenden Höhendifferenz verändert wird. Die zu überwindende Höhendifferenz ist diejenige Höhe eines Prothesenfußes oder eines Fußteils oder eines Fußes eines Nutzers des künstlichen Kniegelenkes zu einem Fuß oder einem Fußteil der kontralateralen Seite des Patienten in dessen Standphase oder die Höhendifferenz zu dem Niveau der unmittelbar vorgehenden Standphase des Prothesenfußes, des Orthesenfußteils oder des Fußes beim Gehen. Neben dem Gehen in der Ebene beinhaltet die übliche Fortbewegung das Gehen auf Rampen, wobei die Rampen sowohl aufwärts als auch abwärts begangen werden, sowie das Gehen auf Treppen, wobei sich insbesondere das Treppe abwärts Gehen von dem abwärts Gehen auf Rampen unterscheidet. Es ist vorgesehen, die maximal mögliche Standphasenbeugung nach dem initialen Fersenkontakt auf einen einstellbaren Winkel zu begrenzen. Eine Standphasenbeugung nach dem initialen Fersenkontakt wird zugelassen, um die direkte Kraftdurchleitung in das Becken des Nutzers zu vermeiden. Dabei wird die Flexionsdämpfung in Abhängigkeit von dem Kniebeugewinkel erhöht, bis ein Zielwinkel erreicht ist oder zumindest nicht überschritten wird. Wird ein Zielwinkel in der Standphase erreicht, wird eine weitere Flexion gesperrt. Bis dahin wird bei einem zunehmenden Flexionswinkel oder Kniebeugewinkel, der sich in einer Verringerung des Kniewinkels auf der posterioren Seite des Kniegelenkes ausdrückt, erhöht, sodass bei Belastungen und Flexionsmomenten, die nicht zu einer weiteren Standphasenflexion bis zu dem maximal erlaubten Zielwinkel führen, keine Sperrung der Flexionsbewegung erfolgt. Dabei wird der zeitliche Verlauf der Flexionswiderstandserhöhung und/oder der maximal erreichbare Flexionswinkel in Abhängigkeit von der Neigung des Untergrundes oder, bei einer Gangsituation des Treppensteigens bei einer zu überwindenden Höhendifferenz verändert. Dadurch ist es möglich, dass der Nutzer während der Standphase eine Kniebeugung oder Flexion durchführen kann, ohne Gefahr zu laufen, durch eine zu hohe Standphasenflexion nicht mehr in eine vollständige Streckung in der Standphase zu kommen. Eine aufwendige Stumpfkontrolle oder ein bewusster Einsatz von noch vorhandener Muskulatur ist nicht mehr notwendig. Dies ermöglicht dem Anwender ein sehr entspanntes und kraftsparendes Gehen, insbesondere bei niedrigen Gehgeschwindigkeiten.

Eine Weiterbildung der Erfindung sieht vor, dass der maximal erreichbare Flexionswinkel und/oder der Flexionswinkel, bei dem der maximale Flexionswiderstand erreicht wird, bei zunehmend abschüssigem Untergrund vergrößert wird. Beim Abwärtsgehen ist es notwendig, das Körpergewicht beim Auftreten mit der versorgten Seite über eine Standphasenflexion abzufangen. Dies wird dadurch erleichtert, dass bei einem zunehmend abschüssigen Untergrund, also wenn es steiler bergab geht, der maximal erreichbare Flexionswinkel vergrößert wird, wodurch ein längerer Weg zum Bereitstellen einer ausreichenden Nachgiebigkeit und ein längerer Weg zum Umwandeln der Bewegungsenergie in Wärme oder elektrische Energie oder in einen anderen Energiespeicher bereitgestellt wird. Darüber hinaus kann bei zunehmend abschüssigem Untergrund der maximale Flexionswiderstand verringert werden, um eine weitere Einbeugung und eine Vergrößerung des maximal erreichbaren Flexionswinkels zu ermöglichen. Ein solch größerer Flexionswinkel kann neben dem Gehen auf einem abschüssigen Untergrund auch bei sogenannten Bremsschritten auftreten oder beim Treppenabwärtsgehen. Wird der maximal erreichbare Flexionswinkel oder der maximale Flexionswiderstand erreicht, bildet sich im zeitlichen Kniewinkelverlauf ein mehr oder weniger ausgebildetes Plateau, da nach Erreichen des Zielwinkels bzw. kurz vor Erreichen einer Flexionssperre eine weitere Kniebeugung verhindert bzw. erschwert wird.

Die Flexionssperre oder der erhöhte Flexionswiderstand kann über einen definierten Zeitraum in der Plateauphase aufrechterhalten und anschließend der Flexionswiderstand verringert werden. Die anschließende Verringerung des Flexionswiderstandes über einen definierten Zeitraum, der auch durch Erreichen einer Orientierung beispielsweise des Oberteils, des Unterteils oder einer Verbindungslinie zwischen Oberteil und Unterteil im Raum festgelegt werden kann, wird die Flexionsdämpfung wieder abgesenkt, beispielsweise auf ein Anfangsniveau einer Standphasendämpfung. Um dabei so wenig wie möglich Kippmomente in dem Oberteil zu erzeugen, wird die Flexionsdämpfung vorteilhafterweise progressiv reduziert, wobei der Zeitraum der Reduktion abhängig von der Untergrundneigung oder der Orientierung der Komponenten zueinander im Raum sein kann.

Der Flexionswiderstand kann nach der Flexionssperre und dem Erreichen des maximalen Standphasenflexionswinkels oder nach der Flexionswiderstandserhöhung und dem Erreichen eines bei diesem Widerstand erreichbaren Standphasenflexionswinkels verringert werden, wenn ein Maß für die maximale Querkraft im Unterteil einen von der Neigung des Untergrundes abhängigen Grenzwert überschreitet und/oder eine Beinsehne eine von der Neigung des Untergrundes abhängige Vorwärtsneigung überschreitet und/oder ein Maß für das Hüftmoment einen Grenzwert zunächst überschreitet und danach unterschreitet. Ein Maß für die Querkraft innerhalb des Unterteils, beispielsweise an einem Unterschenkelrohr oder einer Unterschenkelschiene, ist ein möglicher Indikator für die momentane Gangphase. Das Maß kann die Querkraft selbst sein, es kann aber auch in Abhängigkeit zu der Querkraft definiert sein und beispielsweise in Bezug auf das bekannt oder über einen Sensor an der Orthese oder Prothese ermittelte Körpergewicht sein. Wenn die Querkraft oder das Maß für die Querkraft einen von der Neigung des Untergrundes abhängigen maximalen Wert überschritten hat, kann eine Reduktion des Flexionswiderstandes eingeleitet werden. Die Querkraft ist dabei eine Kraftkomponente, die senkrecht zur Längserstreckung des Unterteils wirkt, bei einem senkrecht stehenden, durchgestreckten Bein verläuft die Querkraft in der Sagittalebene in Anterior-Posterior-Richtung. Der Querkraftwert kann direkt über einen Querkraftsensor gemessen werden, was in dieser Variante der Erfindung der einzige Kraftsensor ist, der benötigt wird, um das Verfahren durchzuführen.

Alternativ oder ergänzend kann der Flexionswiderstand nach einer Flexionssperre und dem Erreichen des maximalen Standphasenflexionswinkels oder nach der Flexionswiderstandserhöhung und dem Erreichen eines damit möglichen Flexionswinkels wieder verringert werden, wenn eine Beinsehne eine von der Neigung des Untergrundes abhängige Vorwärtsneigung überschreitet. Als Beinsehne wird eine Verbindungslinie zwischen zwei definierten Punkten an dem Oberteil und dem Unterteil bzw. einem sich an dem Unterteil anschließenden Bauteil angesehen. Eine bevorzugte Ausgestaltung sieht vor, dass als Beinsehne die Verbindungslinie zwischen einem Hüftdrehpunkt und einem Fußpunkt verwendet wird. Der Hüftdrehpunkt wird bei Benutzung eines Prothesenkniegelenkes von einem Orthopädietechniker ermittelt und legt die Segmentlänge des Oberschenkels oder Oberteils fest, die als Abstand zwischen der Gelenkachse oder Knieachse und dem Hüftdrehpunkt definiert ist. Die Segmentlänge des Unterteils wird über den Abstand zwischen der Knieachse und einem Fußpunkt definiert. Als Fußpunkt kann beispielsweise die Fußmitte, der Momentanpol einer Abrollbewegung, der Endpunkt der Lotlinie des Unterschenkels auf dem Sohlenniveau des Fußteils, des Prothesenfußes oder auf dem Boden definiert werden, andere bodennahe Punkte sind ebenfalls geeignet, um einen Fußpunkt zu definieren. Da bei Orthesen oder Exoskeletten ein Fußteil zur Auflage eines noch vorhandenen natürlichen Fußes nicht notwendig ist, kann auch der Abstand von dem Boden zur Gelenkachse verwendet werden. Die Lage und/oder die Länge der Beinsehne liefern zuverlässig Informationen über die Orientierung des Beines und den Bewegungsfortschritt. Die Beinsehne kann über Absolutwinkelsensoren in Verbindung mit den bekannten Segmentlängen, einem Absolutwinkelsensor und einem Kniewinkelsensor errechnet oder abgeschätzt werden. Wenn die Beinsehne eine Vorwärtsneigung relativ zu dem Untergrund überschreitet, kann daraus auf einen Bewegungsfortschritt geschlossen werden, der es ermöglicht, die Flexionssperre aufzuheben oder den Flexionswiderstand weiter zu verringern, damit ein Durchschwingen und eine Einleitung der Schwungphase erreicht werden kann. Mit Überschreiten der Vorwärtsneigung in Abhängigkeit von der Neigung des Untergrundes werden auch die mittlere Standphase und das Ende der mittleren Standphase detektiert. Die Neigung des Untergrundes kann beispielsweise aus einem ermittelten Winkel im Knöchelgelenk erhalten werden. Die Untergrundneigung kann aber auch auf andere Art und Weise ermittelt werden.

Alternativ oder ergänzend kann der Flexionswiderstand nach der Flexionssperre und dem Erreichen des maximalen Standphasenflexionswinkels oder nach der Flexionswiderstandserhöhung und dem Erreichen eines bei diesem Widerstand erreichbaren Standphasenflexionswinkels verringert werden, wenn ein Maß für das Hüftmoment einen Grenzwert zunächst überschreitet und danach wieder unterschreitet. Das Überschreiten und Unterschreiten erfolgen in einer einzigen Standphase. Das Auftreten eines hohen Hüftmomentes in der Standphase ist ein Indikator für die Neigung des Untergrundes, da sich beispielsweise bei einem Gehen auf einer steilen Rampe bergauf zunächst ein hohes flektierendes Hüftmoment einstellt, dass sich im weiteren Verlauf des Schrittes verringert. Gleiches gilt für das aufwärts Gehen auf einer Treppe. Wird ein extendierendes Hüftmoment detektiert, ist dieses ein Indikator für ein bergab Gehen auf einer Rampe. Das extendierende Hüftmoment verringert sich im Verlauf des Schrittes in der Standphase, so dass bei einem anfänglichen Überschreiten eines Grenzwertes und einem darauf folgenden Unterschreiten auf die Neigung des Untergrundes geschlossen werden kann, um den Flexionswiderstand entsprechend anzupassen. Das Hüftmoment kann über ein Kniemoment und die bekannten geometrischen Beziehungen, über die Orientierung des Oberteils im Raum oder aus der Orientierung des Unterteils im Raum und dem Kniewinkel errechnet werden. Als Maß für das Hüftmoment kann neben dem Hüftmoment als solchem auch eine darauf bezogene Größe verwendet werden, zum Beispiel ein Wert oder eine Kennziffer, die in Abhängigkeit von der Raumlage von Oberteil und/oder Körpergewicht gebildet wird.

Alternativ zu einer direkten Messung der Querkräfte ist es möglich, die das Maß für die Querkraft aus einer Differenz von Querkraftkomponenten eines Knöchelmomentes und eines Kniemomentes zu ermitteln. Wird dazu noch das Körpergewicht des Nutzers des künstlichen Kniegelenkes berücksichtigt, kann eine besonders individuelle Anpassung der Steuerung und der Flexionswiderstände erfolgen.

Der Flexionswiderstand kann nach einer Erhöhung wieder verringert werden, wenn ein vorgegebener Kniebeugewinkel überschritten wird, wobei die Verringerung auf ein Niveau unterhalb eines Sperrniveaus verringert wird. Die Verringerung kann beispielsweise auf ein anfängliches Standphasendämpfungsniveau reduziert werden, wobei der Kniebeugewinkel insbesondere auf steileren Rampen überschritten werden kann, da das Ausmaß der Flexionsdämpfungserhöhung abhängig von der Neigung des Untergrundes ist.

Die Verringerung des Flexionswiderstandes in Abhängigkeit von der Querkraft wird insbesondere bei Bremsschritten relevant, insbesondere bei Bremsschritten in der Ebene und beim Abwärtsgehen von Rampen oder Treppen. Der rampenneigungsabhängige Beinsehnenwinkel oder die Vorwärtsneigung der Beinsehne in Abhängigkeit von der Neigung der Rampe ist insbesondere bei flachen Rampen oder Rampen mit einer mittleren Steigung maßgeblich, um eine zu große Hüftextension zu vermeiden und ein rechtzeitiges Einbeugen des Kniegelenkes zu ermöglichen.

Die Neigung des Untergrundes kann aus einer in der vorhergehenden Schwungphase zurückgelegten vertikalen und/oder horizontalen Distanz des Kniegelenkes, insbesondere eines Referenzpunktes in Fußsohlennähe oder aus dem Verhältnis einer in der vorhergehenden Schwungphase zurückgelegten vertikalen und horizontalen Distanz des Kniegelenkes, aber insbesondere eines Referenzpunktes in Fußsohlennähe, als Wegberechnungskriterium errechnet werden. Dazu werden beispielsweise Sensorsignale einer Inertial Measurement Unit über einen definierten Zeitraum ausgewertet und integriert. Daraus ergeben sich Geschwindigkeiten und zurückgelegte Distanzen, die zur Berechnung der Untergrundneigung genutzt werden können. Die Untergrundneigung ist das Verhältnis aus zurückgelegter vertikaler Distanz zu zurückgelegter horizontaler Distanz. Dabei muss der zurückgelegte Weg eines Punktes in Fußsohlennähe berechnet werden, also der zurückgelegte Weg eines Referenzpunktes. Dazu wird die Lage des Unterteils oder Unterschenkelteils zu Beginn und am Ende der Integration ermittelt und über geometrische Größen und eine vereinfachte Winkelfunktion die zurückgelegte Distanz des Referenzpunktes oder des Fußes relativ zu der Inertial Measurement Unit oder IMU berechnet.

Der Beginn der zu steuernden Standphase kann anhand eines Axialkraftimpulses, einer Plantarflexionsbeschleunigung und/oder eines Knöchelmomentes bestimmt werden. Über einen reinen Axialkraftsensor in einem Fußteil oder an dem Unterteil kann ermittelt werden, wann ein Fuß aufsetzt. Nach einer kraftlosen Phase oder einer Phase ohne Axialkraft wird ein spontaner Anstieg einer Axialkraftkomponente detektiert und dient als aussagekräftiger Indikator für den Beginn der Standphase. Ohne einen Kraftsensor kann eine Plantarflexionsbeschleunigung ermittelt werden, wenn es sich um ein gelenkiges Fußteil oder einen gelenkig gelagerten Prothesenfuß an dem Unterteil handelt. Ebenso kann ein Knöchelmoment, was in Plantarflexionsrichtung wirkt, ermittelt und nach einer momentenlosen Phase eine Plantarflexion bewirkend als Startpunkt für die zu steuernden Standphase genutzt werden.

Eine Variante der Erfindung sieht vor, dass die Neigung des Untergrundes aus einer Auswertung des Flexionswinkels und eines Absolutwinkels eines Oberteils oder eines Unterteils oder aus der Auswertung zweier Absolutwinkel von Oberteil und Unterteil als ein kinematisches Kriterium errechnet wird. Der Verlauf des Kniewinkels wird erfasst und zusammen mit einem Absolutwinkel eines Oberteils oder eines Unterteils ermittelt. Alternativ wird der Absolutwinkel von Oberteil und Unterteil als kinematisches Kriterium verwendet und daraus die Neigung des Untergrundes errechnet. Nach dem Auftreten oder dem initialen Fersenkontakt ergeben sich in Abhängigkeit von der Untergrundneigung unterschiedliche Tangentensteigungen zwischen der Neigung des Unterteils und dem Kniewinkel, sodass in Kenntnis der jeweiligen Tangentensteigung auf die jeweilige Untergrundneigung rückgeschlossen werden kann. Dazu kann die Kniewinkelgeschwindigkeit und die Unterteilwinkelgeschwindigkeit im Raum während des Gehens ermittelt werden. Daraus wird der Quotient der beiden Winkelgeschwindigkeiten errechnet, wobei die Neigung des Untergrundes anhand der Änderungen des Quotienten der Winkelgeschwindigkeiten ermittelt wird.

Ein solches kinematisches Kriterium oder eine solche auf kinematischen Größen basierende Berechnung der Neigung des Untergrundes kann mit dem Wegberechnungskriterium über die Errechnung der zurückgelegten vertikalen und/oder horizontalen Distanz gemeinsam verwendet werden, wobei eine gewichtete Verwendung der jeweiligen Kriterien möglich ist. Neben der gleichgewichteten Berücksichtigung der errechneten Neigung aus der Bewegung von Unterschenkel und Oberschenkel und der errechneten Neigung aufgrund der Wegberechnungsdaten aus den Signalen einer IMU kann beispielsweise das kinematische Kriterium geringer gewichtet werden oder nur in bestimmten Situationen oder Gangsituationen als zusätzliche Bestimmungsgröße oder Fehlervermeidungsmaßnahme eingesetzt werden. Beispielsweise kann in kritischen Situationen bei dem Treppe abwärts Gehen zusätzlich zu dem Wegberechnungskriterium das kinematische Kriterium dazu dienen, ein unbeabsichtigtes Sperren oder Freigeben des Kniegelenkes zu vermeiden.

Als eine weitere Steuergröße kann die Lage und/oder die Orientierung eines Bodenreaktionskraftvektors in Bezug auf die Prothese oder Orthese verwendet werden. Ebenso ist es möglich, dass das Erkennen eines Überrollens eines Fußteils über eine Kante eine Dämpfungserhöhung verhindert oder den erhöhten Widerstand weiter reduziert, was insbesondere bei einem Treppe abwärts Gehen beim Abrollen des versorgten Beines vorteilhaft ist. Die zurückgelegten Distanzen für das Wegberechnungskriterium werden insbesondere aus den IMU-Werten des Unterteils zum Ende der vorhergehenden Standphase und zu Beginn der zu steuernden Standphase berechnet, wobei die Entfernung zwischen der Position der IMU an der Orthese oder Prothese zu dem jeweiligen Referenzpunkt sowie die Raumlagen am Ende der Standphase, also beim toe-off und beim initialen Fersenkontakt oder heel strike bekannt sind. Sowohl das Wegberechnungskriterium als auch das kinematische Kriterium können einzeln zur Bestimmung der Untergrundneigung verwendet werden, wobei auch die Trennschärfe der jeweiligen Sensorsignale ein Faktor für die Anwendung des einen Kriteriums oder des anderen Kriteriums sein kann.

Der Startpunkt und der Endpunkt der Wegintegration können über einen Zustandsautomaten bestimmt werden, wobei verschiedene Sensorsignale auf verschiedene Ereignisse hin überwacht werden. Ein solches Ereignis wäre beispielsweise ein belastetes Überrollen auf einer Treppenkante, was durch eine Erfassung einer Axialkraft bei einer gleichzeitigen Vorwärtsneigung zumindest des Unterteils oder der Beinsehne erkannt werden kann. Ebenso können ein belastetes Überrollen oder ein Anheben der Orthese oder Prothese sowie ein Wiederbelasten der Orthese oder Prothese als entscheidendes Merkmal für den Start- und Endzeitpunkt der Wegintegration dienen.

Nachfolgend werden Ausführungsbeispiele der Erfindung anhand der beigefügten Figuren näher erläutert. Es zeigen:
- Figur 1 -: eine schematische Darstellung eines Prothesenbeines;
- Figur 2 -: eine Darstellung von Beinsehnen;
- Figur 3 -: eine Definition eines Höhenunterschiedes beim Gehen;
- Figur 4 -: eine Darstellung einer neigungsabhängigen Einstellung von Flexionswinkel und Flexionswiderstand;
- Figur 5a - 5c: -unterschiedliche Verläufe von Flexionswinkel und Flexionswiderstand bei unterschiedlichen Untergrundneigungen;
- Figur 6 -: Darstellungen von Flexionswinkel und Rollwinkel bei unterschiedlichen Untergrundneigungen;
- Figur 7-: Darstellungen eines kinematischen Kriteriums bei unterschiedlichen Untergrundneigungen;
- Figur 8 -: eine Darstellung eines geometrischen Kriteriums beim Abwärtsgehen auf einer Rampe; sowie
- Figur 9 -: eine Darstellung einer Orthese.

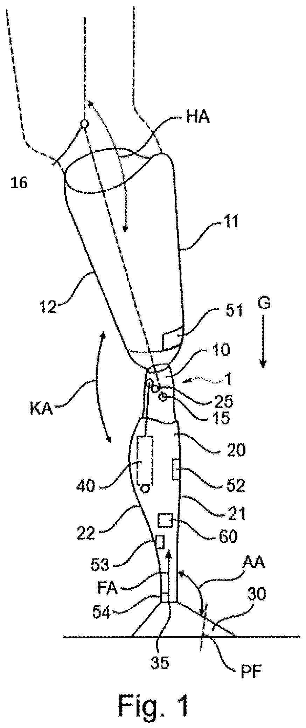

Figur 1 zeigt eine schematische Darstellung eines künstlichen Kniegelenkes 1 in einer Anwendung an einem Prothesenbein. Alternativ zu einer Anwendung an einem Prothesenbein kann ein entsprechend ausgestaltetes künstliches Kniegelenk 1 auch in einer Orthese oder einem Exoskelett eingesetzt werden. Statt eines Ersatzes eines natürlichen Gelenkes wird dann das jeweilige künstliche Kniegelenk medial und/oder lateral an dem natürlichen Gelenk angeordnet. Im dargestellten Ausführungsbeispiel ist das künstliche Kniegelenk 1 in Gestalt eines Prothesenkniegelenkes mit einem Oberteil 10 mit einer anterioren oder in Gehrichtung gelegenen oder vorderen Seite 11 und einer posterioren Seite 12, die der anterioren Seite 11 gegenüberliegt, ausgebildet. An dem Oberteil 10 ist ein Unterteil 20 schwenkbar um eine Schwenkachse 15 gelagert. Auch das Unterteil 20 weist eine anteriore Seite 21 oder vordere Seite und eine posteriore Seite 22 oder hintere Seite auf. In dem dargestellten Ausführungsbeispiel ist das Kniegelenk 1 als ein monozentrisches Kniegelenk ausgebildet, grundsätzlich ist es auch möglich, ein polyzentrisches Kniegelenk entsprechend zu steuern. An dem distalen Ende des Unterteils 20 ist ein Fußteil 30 angeordnet, das entweder als ein starres Fußteil 30 mit einem unbeweglichen Fußgelenk oder aber mit einer Schwenkachse 35 mit dem Unterteil verbunden sein kann, um einen an den natürlichen Bewegungsablauf angenäherten Bewegungsablauf zu ermöglichen.

Zwischen der posterioren Seite 12 des Oberteils 10 und der posterioren Seite 22 des Unterteils 20 wird der Kniewinkel KA gemessen. Der Kniewinkel KA kann über einen Kniewinkelsensor 25, der im Bereich der Schwenkachse 15 angeordnet sein kann, direkt gemessen werden. Der Kniewinkelsensor 25 kann mit einem Momentensensor gekoppelt sein oder einen solchen aufweisen, um ein Kniemoment um die Gelenkachse 15 zu erfassen. An dem Oberteil 10 ist ein Inertialwinkelsensor oder eine IMU 51 angeordnet, der die Raumlage des Oberteils 10 misst, beispielsweise im Verhältnis zu einer konstanten Kraftrichtung, beispielsweise der Gravitationskraft G, die vertikal nach unten zeigt. An dem Unterteil 20 ist ebenfalls ein Inertialwinkelsensor oder eine IMU 53 angeordnet, um die Raumlage des Unterteils während der Benutzung des Prothesenbeines zu ermitteln.

Zusätzlich zu dem Inertialwinkelsensor 53 kann an dem Unterteil 20 oder dem Fußteil 30 ein Beschleunigungssensor und/oder Querkraftsensor 53 angeordnet sein. Über einen Kraftsensor oder Momentensensor 54 an dem Unterteil 20 oder Fußteil 30 kann eine auf das Unterteil 20 wirkende Axialkraft FA oder ein um die Knöchelgelenksachse 35 wirkendes Knöchelmoment ermittelt werden kann.

Zwischen dem Oberteil 10 und dem Unterteil 20 ist eine Widerstandseinrichtung 40 angeordnet, um eine Verschwenkbewegung des Unterteils 20 relativ zu dem Oberteil 10 zu beeinflussen. Die Widerstandseinrichtung 40 kann als passiver Dämpfer, als Antrieb oder als ein sogenannter semiaktiver Aktuator ausgebildet sein, mit dem es möglich ist, Bewegungsenergie zu speichern und zu einem späteren Zeitpunkt gezielt wieder abzugeben, um Bewegungen abzubremsen oder zu unterstützen. Die Widerstandseinrichtung 40 kann als lineare oder rotatorische Widerstandseinrichtung ausgebildet sein. Die Widerstandseinrichtung 40 ist mit einer Steuerungseinrichtung 60 verbunden, beispielsweise verkabelt oder über eine drahtlose Verbindung, die wiederum mit zumindest einem der Sensoren 25, 51, 52, 53, 54 gekoppelt ist. Die Steuerungseinrichtung 60 verarbeitet die von den Sensoren übermittelten Signale elektronisch mit Prozessoren, Recheneinheiten oder Computern. Sie weist eine elektrische Energieversorgung sowie zumindest eine Speichereinheit auf, in der Programme und Daten gespeichert sind und in der ein Arbeitsspeicher zur Verarbeitung von Daten bereit steht. Nach der Verarbeitung der Sensordaten erfolgt die Ausgabe eines Aktivierungs- oder Deaktivierungsbefehles, mit dem die Widerstandseinrichtung 40 aktiviert oder deaktiviert wird. Durch Aktivieren eines Aktuators in der Widerstandseinrichtung 40 kann beispielsweise ein Ventil geöffnet oder geschlossen oder ein Magnetfeld erzeugt werden, um ein Dämpfungsverhalten zu verändern.

An dem Oberteil 10 des Prothesenkniegelenkes 1 ist ein Prothesenschaft befestigt, der zur Aufnahme eines Oberschenkelstumpfes dient. Über den Oberschenkelstumpf ist das Prothesenbein mit dem Hüftgelenk 16 verbunden. Auf der anterioren Seite des Oberteils 10 wird ein Hüftwinkel HA gemessen, der zwischen einer vertikalen Linie durch das Hüftgelenk 16 und der Längserstreckung des Oberteils 10 und der Verbindungslinie zwischen dem Hüftgelenk 16 und der Kniegelenksachse 15 auf der anterioren Seite 11 angetragen ist. Wird der Oberschenkelstumpf angehoben und das Hüftgelenk 16 flektiert, verringert sich der Hüftwinkel HA, beispielsweise beim Hinsetzen. Umgekehrt vergrößert sich der Hüftwinkel HA bei einer Extension, beispielsweise beim Aufstehen oder ähnlichen Bewegungsabläufen.

Während eines Gangzyklusses beim Gehen in der Ebene wird das Fußteil 30 zunächst mit der Ferse aufgesetzt, der erste Kontakt der Ferse oder eines Fersenteils des Fußteils 30 wird Heel Strike genannt. Anschließend erfolgt eine Plantarflexion, bis das Fußteil 30 vollständig auf dem Boden aufliegt, in der Regel ist dabei die Längserstreckung des Unterteils 10 hinter der Vertikalen, die durch die Knöchelgelenksachse 35 verläuft. Während des Gehens in der Ebene wird dann der Körperschwerpunkt nach vorne verlagert, das Unterteil 20 verschwenkt nach vorne, der Knöchelwinkel AA verkleinert sich und es findet eine zunehmende Belastung des Vorderfußes statt. Der Bodenreaktionskraftvektor wandert von der Ferse nach vorne bis zum Vorderfuß. Zum Ende der Standphase erfolgt eine Zehenablösung oder der sogenannte Toe-off, danach schließt sich die Schwungphase an, in der das Fußteil 30 beim Gehen in der Ebene unter Verringerung des Kniewinkels KA hinter den Schwerpunkt oder das Hüftgelenk der ipsilateralen Seite verlagert wird, um dann nach Erreichen eines minimalen Kniewinkels KA nach vorne gedreht zu werden, um dann mit einem in der Regel maximal gestreckten Kniegelenk 1 wieder den Fersenkontakt zu erreichen. Der Krafteinleitungspunkt PF wandert somit während der Standphase von der Ferse bis zum Vorderfuß und ist in der Figur 1 schematisch dargestellt.

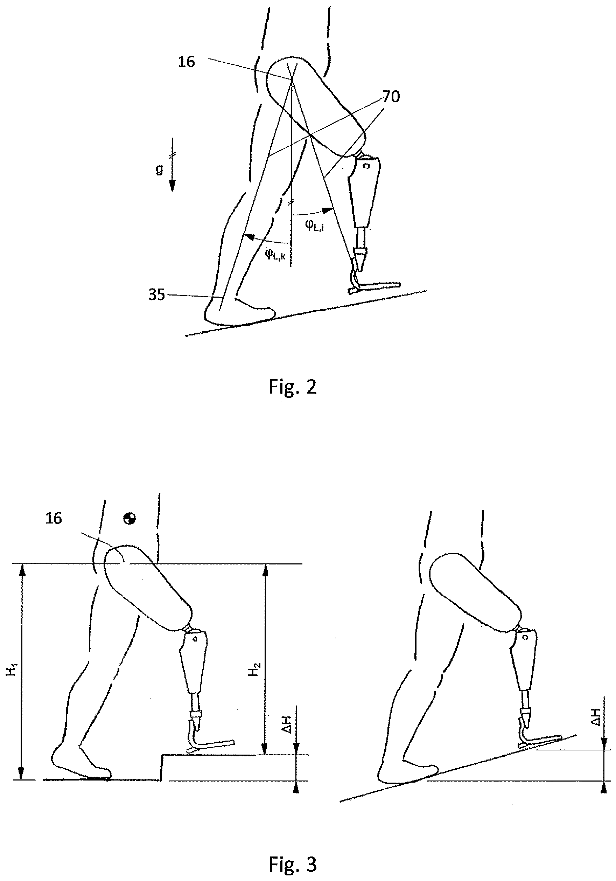

In der Figur 2 ist eine Definition der Beinsehnen 70 eines ipsilateralen, versorgten Beines und eines kontralateralen, unversorgten Beines vorgenommen. Die Beinsehne geht durch den Hüftdrehpunkt 16 und bildet eine Linie zu dem Knöchelgelenk 35. Wie der Figur 2 zu entnehmen ist, verändert sich die Länge der Beinsehne und die Orientierung φ_{L} der Beinsehnen 70 bei der Bewegung, insbesondere auch bei unterschiedlichen Steigungen. Über den Verlauf der Änderung der Länge und/oder Orientierung der Beinsehne 70 können zu überwindendende Höhendifferenzen ΔH abgeschätzt und vorausgesagt oder ermittelt werden. Daraus werden dann die jeweiligen Steuerbefehle abgeleitet. Die jeweilige Orientierung der ipsilateralen Beinsehne φ_{Li} relativ zu der Gravitationsrichtung G als der Senkrechten und der kontralateralen Beinsehne φ_{Lk} ist jeweils eingetragen.

Anhand der Figur 3 kann die Schritthöhe zwischen dem kontralateralen, unversorgten Bein und dem ipsilateralen Fußteil 30 des versorgten Beines definiert werden. Beispielsweise wird der Abstand H₁ von dem Boden bis zu einem markanten Punkt der Hüfte, beispielsweise dem Hüftgelenk 16 oder dem Trochanter major, auf Höhe des Standbeines festgelegt, der Abstand H₂ ist der Abstand zwischen dem Boden und dem Hüftgelenk 16 oder des Trochanter majors auf der vorauseilenden Seite, im dargestellten Beispiel der vorsorgten Seite. Die Höhendifferenz ΔH ergibt sich dann aus der Differenz zwischen H1 und H2. Entsprechend gilt eine Definition der Höhendifferenz ΔH für das Gehen auf einer Rampe.

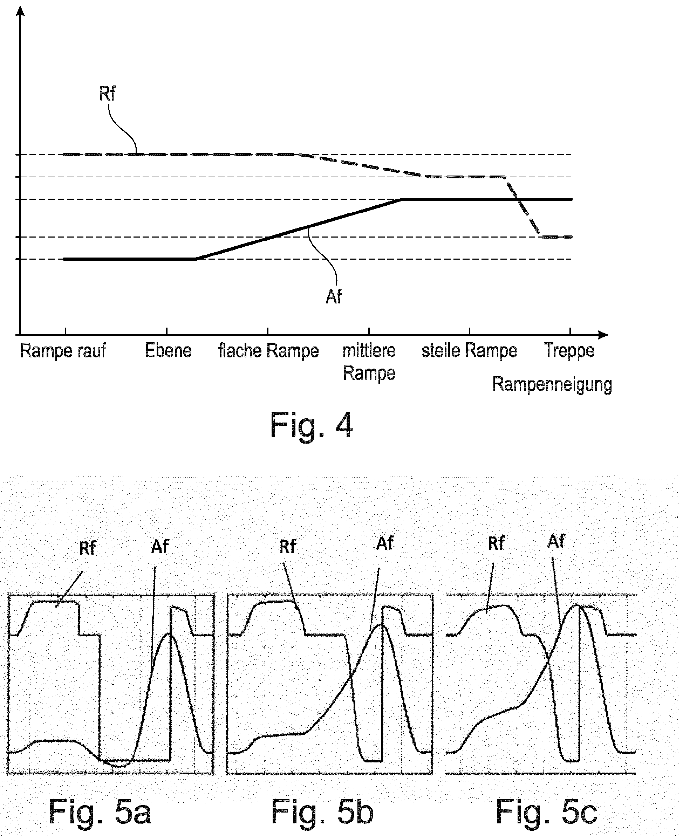

In der Figur 4 ist eine Darstellung unterschiedlicher Einstellwerte eines Flexionswiderstandes Rf und eines Flexionswinkels Af gezeigt. Der Flexionswiderstand Rf sowie der Flexionswinkel Af sind jeweils als maximale Werte eingestellt. Die maximale Flexionsdämpfung oder der maximale Flexionswiderstand Rf bleibt beim Rampe aufwärts Gehen, beim Gehen in der Ebene sowie auf flachen Rampen nahezu konstant. Erst bei zunehmender Abwärtsneigung der Rampen wird der maximale Flexionswiderstand verringert, beispielsweise um 5%. Für die Situation eines Treppe abwärts Gehens wird dann der maximale Flexionswiderstand Rf auf ein wesentlich niedrigeres Niveau verringert, insbesondere auf ein Niveau der Standphasendämpfung für das Gehen in der Ebene. Der maximale Flexionswinkel Af wird ebenfalls in Abhängigkeit von der Neigung des Untergrundes verändert. Das Rampe aufwärts Gehen und das Gehen in der Ebene finden bei einem gleichen maximalen Flexionswinkel Af in der Standphase statt. Der maximal erreichbare Flexionswinkel Af wird in Abhängigkeit von der Untergrundneigung vergrößert, bis auf einen Maximalwert, der für das Gehen auf steilen Rampen und das Treppe abwärts Gehen eingestellt. Eine solche Standphasensteuerung passt den Flexionswiderstand Rf während der frühen und mittleren Standphase in Abhängigkeit der Neigung des Untergrundes an und begrenzt damit auch den maximalen Flexionswinkel innerhalb der Standphase. Der maximal mögliche Flexionswinkel Af ist einstellbar, wobei der Grenzwert durch die Veränderung des Flexionswiderstandes Rf bewirkt wird. In Abhängigkeit von der Untergrundneigung wird ein maximaler Zielwert vorgegeben, mit dem Flexionswiderstand Rf einen so hohen Wert aufweist, dass keine weitere Kniebeugung oder Knieflexion möglich ist. Wird der maximale Flexionswinkel Af in Abhängigkeit von der Untergrundneigung erreicht, bildet sich im weiteren Bewegungsverlauf, also beim Gehen in der Ebene, auf der Rampe oder beim Treppensteigen, ein jeweils unterschiedliches Kniewinkelplateau aus, da eine weitere Flexion verhindert wird. Wird erkannt, dass ein vergrößerter Flexionswinkel notwendig ist, beispielsweise beim Gehen auf mittleren oder steilen Rampen oder beim Treppensteigen, wird der Flexionswiderstand Rf verringert, sodass eine Vergrößerung des Flexionswinkels Af auch ohne Plateau möglich ist. So wird beispielsweise für das Treppensteigen der Flexionswiderstand Rf auf das Standphasendämpfungsniveau reduziert.

In den Figuren 5a bis 5c sind zeitliche Verläufe von Flexionswinkel Af und Flexionswiderstand Rf für unterschiedliche Untergrundneigungen dargestellt. Die linke Darstellung in Figur 5a zeigt ein Gehen in der Ebene, die mittlere Darstellung in Figur 5b den Verlauf der beiden Kenngrößen für das Gehen auf flachen Rampen, das rechte Diagramm in Figur 5c zeigt den Verlauf der Kenngrößen beim Gehen auf Rampen mit einer mittleren Neigung. In der Darstellung der Figur 5a wird nach dem Initialkontakt zunächst eine Knieflexion bewirkt, sodass der Flexionswinkel Af ansteigt. Zusammen mit dem Ansteigen des Flexionswiderstandes Rf wird in dem linken Drittel der Figur 5a deutlich, dass eine weitere Flexion unterbunden ist, sodass ein Plateau sowohl des Flexionswiderstandes Rf als auch des Flexionswinkels Af auftritt. Im Verlauf des weiteren Gangfortschrittes, nach dem Überrollen, verringert sich der Flexionswinkel Af. Anschließend wird ab einem bestimmten Grenzwert der Flexionswiderstand Rf verringert, um am Ende der Standphase eine weitergehende Flexion zu ermöglichen, damit in der Schwungphase ein ausreichend großer Flexionswinkel Af erreicht werden kann.

In der Figur 5b ist das Gehen auf einer flachen Rampe gezeigt. Auch hier wird der Flexionswiderstand Rf nach dem heel strike erhöht, bis eine weitere Flexion und Erhöhung des Flexionswinkels Af nicht mehr möglich ist. An die Plateauphase im ersten Drittel des Diagrammes der Figur 5b schließt sich anders als bei der Figur 5a keine Extension an, sondern ein Überrollen mit einem flektierten Kniegelenk. Ein solcher Bewegungsverlauf ist typisch für das Abwärtsgehen auf einer flachen Rampe. Der Flexionswiderstand Rf wird nach einem definierten Zeitraum reduziert. Der Zeitraum kann beispielsweise so bemessen sein, dass nach dem heel strike und einer normalen Gehgeschwindigkeit ein vollständiger Kontakt mit dem Boden durch das Fußteil erreicht wurde. Es können statistische Daten für die Dauer einer Standphase und damit auch für das erste Absenken des Flexionswiderstandes Rf zur Einleitung einer Einbeugung verwendet werden. Der Flexionswiderstand Rf wird abgesenkt, sodass eine weitere Einbeugung und Erhöhung des Flexionswinkels Af stattfinden kann. Auch hier wird die Schwungphase zugelassen und der Flexionswiderstand Rf auf einen minimalen Wert reduziert.

Die Figur 5c zeigt den Kenngrößenverlauf auf einem steileren Untergrund, die Plateauphase nach dem Erhöhen des Flexionswiderstandes Rf ist sehr kurz, die Verringerung des Flexionswiderstandes Rf zum Durchschwingen und Einleitung der Schwungphase erfolgt wie bei der Figur 5b zu einem spätestmöglichen Zeitpunkt, um eine ausreichende Stabilität insbesondere beim Bergabgehen zu erhalten. Die jeweiligen Abflachungen im Verlauf des Kniewinkels Af gehen einher mit der Verlangsamung der anfänglichen Knieflexion aufgrund des erhöhten Flexionswiderstandes Rf. Dadurch wird beim Durchschwingen der kontralateralen, also unversorgten Seite mehr Bodenfreiheit generiert. Unnötige Ausgleichsbewegungen auf der kontralateralen Seite können dadurch vermieden werden. Von besonderer Bedeutung sind die Verläufe des Flexionswiderstandes Rf zwischen dem heel strike oder initialen Fersenkontakt und einer erneuten Extensionsbewegung oder bei einer Überrollbewegung.

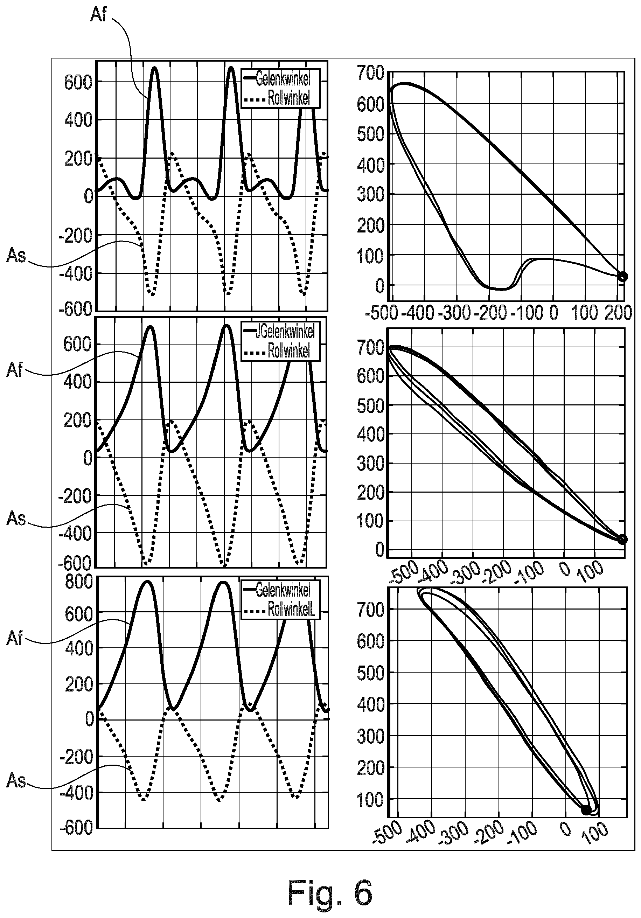

In der Figur 6 sind drei Diagramme für unterschiedliche Untergrundneigungen dargestellt, die obere Darstellung betrifft das Gehen in der Ebene, die mittlere Darstellung betrifft das Rampe abwärts Gehen und die untere Darstellung das Treppe abwärts Gehen. In der jeweils linken Grafik sind die Verläufe des Flexionswinkels Af sowie der Neigungswinkel oder Rollwinkel As des Unterteils dargestellt. In der jeweils rechten Darstellung sind der Rollwinkel As als X-Komponente und der Flexionswinkel Af als Y-Komponente aufgezeichnet. Der jeweilige Fersenkontakt ist mit einem Kreis markiert. Ausgehend von dem Fersenkontakt durchläuft das Diagramm eine geschlossene, zweidimensionale Kurve mit jeweils charakteristischen Kurvenverläufen. Zu ausgewählten Zeitpunkten, beispielsweise in gleichbleibenden, diskreten Zeitabschnitten, wird die Tangentensteigung des Kurvenverlaufes berechnet. Dazu wird laufend das Verhältnis aus Flexionswinkelgeschwindigkeit und Rollwinkelgeschwindigkeit berechnet. Das berechnete Verhältnis wird gegebenenfalls über einen Tiefpassfilter geglättet, wobei erst kurz nach dem Fersenkontakt oder heel strike der Filter eingeschaltet oder initialisiert wird, um zu große Signalstörungen aufgrund des initialen Fersenkontaktes zu vermeiden. Von dem Fersenstoß wird über die beginnende Standphasenflexion der berechnete und gefilterte Wert über eine Interpolationsfunktion mit definierten Stützpunkten einer entsprechenden Rampenneigung zugeordnet. Den Darstellungen der Figur 6 ist zu entnehmen, dass für jede Gangsituation mit unterschiedlichen Neigungen unterschiedliche Tangentensteigungen an der Kurve auftreten, sodass die Tangentensteigung dazu dienen kann, die Untergrundneigung zu bestimmen und eine entsprechende Veränderung des Flexionswiderstandes in Abhängigkeit von der Untergrundneigung einzustellen.

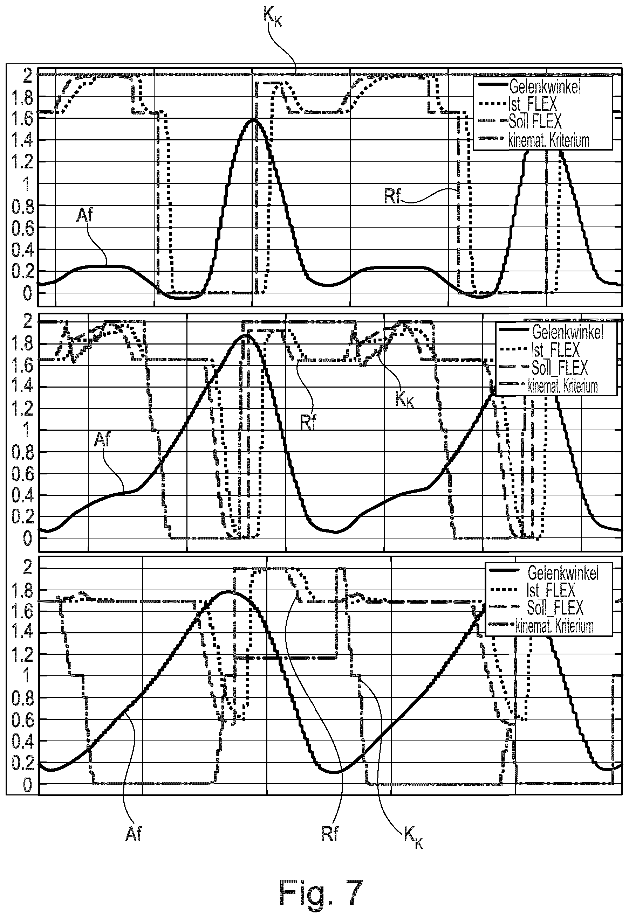

In der Figur 7 sind drei Schaubilder für die Auswertung des kinematischen Kriteriums, also die Auswertung des Flexionswinkels Af und des Rollwinkels As des Unterteils dargestellt. Die obere Grafik stellt das Gehen in der Ebene dar, die mittlere Grafik das Gehen auf einer flachen Rampe, die untere Darstellung ist repräsentativ für das Gehen auf einer steilen Rampe. Der Kurvenverlauf des Flexionswinkels Af entspricht im Wesentlichen dem Verlauf des Flexionswinkels der Figuren 5a bis 5c. Der jeweilige Verlauf des Flexionswiderstandes Rf in der durchgezogenen Linie, die strichlierte Linie ist der zeitversetzte tatsächliche Istwert. Das kinematische Kriterium Kk ist in der oberen Darstellung durchgängig auf dem Wert 2 eingestellt, was dem Gehen in der Ebene entspricht. Der Wert 1 entspricht dem Gehen auf einer mittleren Rampe, der Wert 0 auf einer steilen Rampe oder einer Treppe. In der oberen Darstellung ist zu erkennen, dass das kinematische Kriterium Kk für das Gehen in der Ebene durchgängig korrekte Werte anzeigt und eine entsprechend angepasste Steuerung des Flexionswiderstandes Rf für die Standphasenflexion mit der ausgeprägten Plateauphase für den Flexionswinkel Af bereitstellt. In der mittleren Grafik ist zu sehen, dass der Wert für das kinematische Kriterium Kk auf ungefähr 1,6 sinkt, was einem Gehen auf einer flachen Rampe entspricht. Sobald der Wert des Flexionswiderstandes Rf steigt, tritt ein Selbstverstärkungsprozess ein, der das kinematische Kriterium Kk wieder in Richtung auf das Gehen in der Ebene anhebt. Maßgeblich für die Steuerung des Flexionswiderstandes Rf ist daher das erreichte Minimum des kinematischen Kriteriums Kk nach dem Fersenauftritt. In dem vorliegenden Fall führt dies zu einem Anstieg des Flexionswiderstandes Rf bis zu einer Sperre bei einem signifikant größeren Flexionswinkel Af als beim Gehen in der Ebene.

In der unteren Darstellung der Figur 7 ist zu erkennen, wie das kinematische Kriterium Kk beim Gehen auf sehr steilen Rampen, was mit dem Gehen auf Treppen vergleichbar ist, schnell auf den Wert 0 abfällt. Dadurch wird eine Erhöhung des Flexionswiderstandes Rf vermieden, sodass eine weitergehende Flexion des Knies und eine Erhöhung des Flexionswinkels Af ohne Plateauphase möglich sind. Der jeweils beschrittene Untergrund oder die jeweilige erkannte Gangsituation führt somit zu signifikant unterschiedlichen Veränderungen des Flexionswiderstandes Rf.

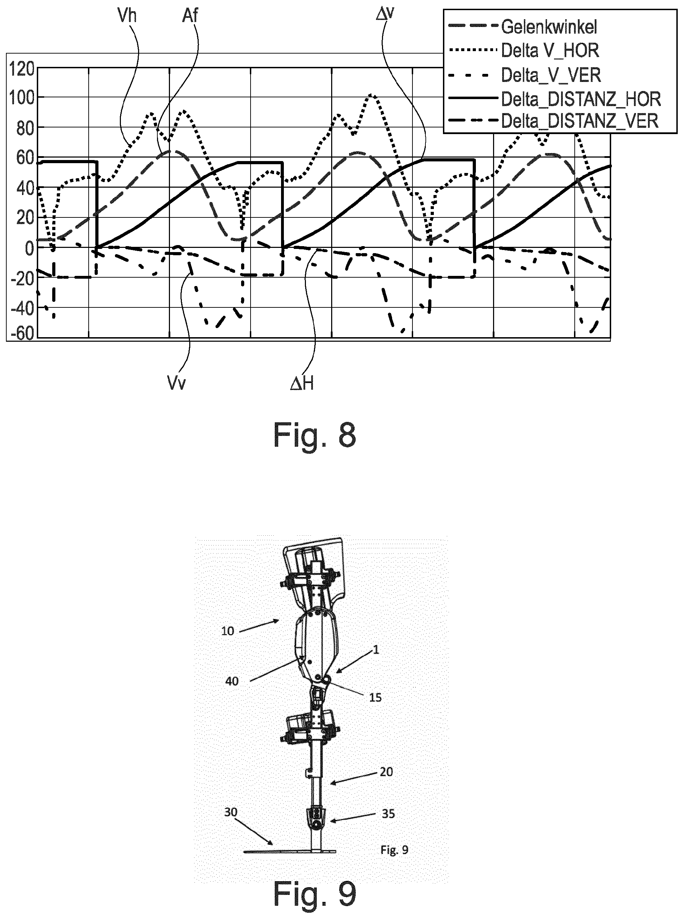

In der Figur 8 ist das Wegberechnungskriterium dargestellt, das basierend auf den Sensorsignalen der IMU über einen definierten Zeitraum ermittelt wird. Dabei werden die Beschleunigungen an dem Kniegelenk 1 oder dem Unterteil 20 in der Vorwärtsrichtung und Aufwärtsrichtung integriert. Die Aufwärtsrichtung wirkt entgegen der Gravitationsrichtung, die Vorwärtsrichtung ist eine Vorwärtsbewegung in Sagittalebene von posterior nach anterior. Durch eine einfache Integration über die Zeit ergeben sich die jeweiligen Geschwindigkeiten, durch eine zweifache Integration über die Zeit der Werte der IMU ergeben sich die jeweils zurückgelegten Distanzen in Vertikalrichtung und Horizontalrichtung. In der Figur 8 sind die zurückgelegten horizontalen Distanzen Δ*V* bezeichnet, die zurückgelegten vertikalen Distanzen sind mit Δ*H* bezeichnet. Die vertikalen Geschwindigkeiten sind mit Vv beschrieben, die horizontalen Geschwindigkeiten mit Vh. In der Figur 8 ist das Gehen auf einer abwärts geneigten Rampe gezeigt. Die globale Untergrundneigung ist definiert als das Verhältnis aus der zurückgelegten horizontalen Distanz Δ*V* zu der zurückgelegten vertikalen Distanz Δ*H* , wobei der zurückgelegte Weg jeweils in Fußsohlennähe berechnet wird. Dazu wird die Lage des Unterteils 2 zu Beginn und am Ende der Integration ermittelt und über die bekannten geometrischen Größen wie Unterteillänge, Position der IMU am Unterteil 20 oder am Oberteil 10 sowie des Kniewinkels lässt sich der zurückgelegte Weg des Fußes relativ zu der jeweiligen IMU berechnen. Auch mit dem Wegberechnungskriterium ist es möglich, mit einer vergleichsweise guten Auflösung die jeweilige Untergrundneigung zu erkennen. Der über das Wegkriterium ermittelte Wert für die globale Untergrundneigung kann dann als Steuerparameter für die Einstellung des Flexionswiderstandes Rf verwendet werden.

In der Figur 9 ist in einer schematischen Darstellung ein Ausführungsbeispiel einer Orthese mit einem Oberteil 10 und einem schwenkbar um eine Schwenkachse 15 daran gelagerten Unterteil 20 gezeigt, mit der das Verfahren ebenfalls ausgeführt werden kann. Zwischen dem Oberteil 10 und dem Unterteil 20 ist dadurch ein künstliches Kniegelenk 1 ausgebildet, das in dem dargestellten Ausführungsbeispiel lateral zu einem natürlichen Kniegelenk angeordnet ist. Neben einer einseitigen Anordnung von Oberteil 10 und Unterteil 20 relativ zu einem Bein können auch zwei Oberteile und Unterteile medial und lateral zu einem natürlichen Bein angeordnet sein. Das Unterteil 20 weist an seinem distalen Ende ein Fußteil 30 auf, das um eine Knöchelgelenksachse 35 schwenkbar zu dem Unterteil 20 gelagert ist. Das Fußteil 30 weist eine Fußplatte auf, auf der ein Fuß oder Schuh aufgesetzt werden kann. Sowohl an dem Unterteil 20 als auch an dem Oberteil 30 sind Befestigungseinrichtungen zum Festlegen an dem Unterschenkel bzw. Oberschenkel angeordnet. An dem Fußteil 30 können auch Einrichtungen zum Festlegen des Fußes auf dem Fußteil 30 angeordnet sein. Die Befestigungseinrichtungen können als Schnallen, Gurte, Spangen oder dergleichen ausgebildet sein, um die Orthese lösbar an dem Bein des Nutzers anlegen und zerstörungsfrei wieder abnehmen zu können. An dem Oberteil 10 ist die Widerstandseinrichtung 40 befestigt, die sich an dem Unterteil 20 und an dem Oberteil 10 abstützt und einen einstellbaren Widerstand gegen eine Verschwenkung um die Schwenkachse 15 bereitstellt. Die Sensoren und die Steuerungseinrichtung, die weiter oben im Zusammenhang mit dem Ausführungsbeispiel der Prothese beschrieben wurden, sind entsprechend auch an der Orthese vorhanden.

## Patentansprüche

1. Verfahren zur Steuerung einer Prothese oder Orthese der unteren Extremität mit einem Oberteil (10) und einem mit dem Oberteil (20) über ein Kniegelenk (1) verbundenes Unterteil (20), das um eine Gelenkachse (15) relativ zu dem Oberteil (10) verschwenkbar gelagert ist, wobei zwischen dem Oberteil (10) und dem Unterteil (20) eine verstellbare Widerstandseinrichtung (40) angeordnet ist, über die beim Gehen auf der Grundlage von Sensordaten ein Flexionswiderstand (Rf) in einer frühen und mittleren Standphase nach einem initialen Fersenkontakt bis zu der mittleren Standphase verändert wird, **dadurch gekennzeichnet, dass** nach dem initialen Fersenkontakt der Flexionswiderstand (Rf) auf einen Wert erhöht wird, bei dem eine weitere Flexion gesperrt oder zumindest verlangsamt wird, wobei der zeitliche Verlauf der Flexionswiderstandserhöhung und/oder der maximal erreichbare Flexionswinkel (Af) in Abhängigkeit von der Neigung des Untergrundes oder einer zu überwindenden Höhendifferenz (ΔH) verändert wird.

2. Verfahren nach Anspruch 1, **dadurch gekennzeichnet, dass** der maximal erreichbare Flexionswinkel (Af) und/oder der Flexionswinkel (Af), bei dem der maximale Flexionswiderstand (Rf) erreicht wird, bei zunehmend abschüssigen Untergrund vergrößert wird.

3. Verfahren nach Anspruch 1 oder 2, **dadurch gekennzeichnet, dass** bei zunehmend abschüssigen Untergrund der maximale Flexionswiderstand (Rf) verringert wird.

4. Verfahren nach einem der voranstehenden Ansprüche, **dadurch gekennzeichnet, dass** die Flexionssperre oder die Flexionswiderstandserhöhung über einen definierten Zeitraum aufrechterhalten wird und anschließend der Flexionswiderstand (Rf) verringert wird.

5. Verfahren nach Anspruch 4, **dadurch gekennzeichnet, dass** der Flexionswiderstand nach der Flexionssperre oder der Flexionswiderstandserhöhung verringert wird, wenn ein Maß für die Querkraft im Unterteil (20) einen von der Neigung des Untergrundes abhängigen Grenzwert überschreitet und/oder eine Beinsehne (70) eine von der Neigung des Untergrundes abhängige Vorwärtsneigung überschreitet und/oder ein Maß für das Hüftmoment einen Grenzwert zunächst überschreitet und danach unterschreitet.

6. Verfahren nach Anspruch 5, **dadurch gekennzeichnet, dass** das Maß für die Querkraft über einen Querkraftsensor oder aus einer Differenz von Querkraftkomponenten eines Knöchelmomentes und Kniemomentes ermittelt wird.

7. Verfahren nach einem der voranstehenden Ansprüche, **dadurch gekennzeichnet, dass** der Flexionswiderstand (Rf) nach einer Erhöhung auf unterhalb eines Sperrniveaus verringert wird, wenn ein vorgegebener Flexionswinkel (Af) überschritten wird.

8. Verfahren nach einem der voranstehenden Ansprüche, **dadurch gekennzeichnet, dass** die Neigung des Untergrundes aus einer in der vorhergehenden Schwungphase zurückgelegten vertikalen und/oder horizontalen Distanz des Kniegelenkes (1), insbesondere eines Referenzpunktes in Fußsohlennähe oder aus dem Verhältnis einer in der vorhergehenden Schwungphase zurückgelegten vertikalen und horizontalen Distanz des Kniegelenkes (1), insbesondere eines Referenzpunktes in Fußsohlennähe, als Wegberechnungskriterium errechnet wird.

9. Verfahren nach Anspruch 8, **dadurch gekennzeichnet, dass** der Beginn der zu steuernden Standphase anhand eines Axialkraftimpulses, einer Plantarflexionsbeschleunigung und/oder eines Knöchelmomentes bestimmt wird.

10. Verfahren nach einem der Ansprüche 1 bis 7, **dadurch gekennzeichnet, dass** die Neigung des Untergrundes aus einer Auswertung eines Flexionswinkels (Af) und einem Absolutwinkel des Oberteils (10) oder des Unterteils (20) oder aus der Auswertung zweier Absolutwinkel von Oberteil (10) und Unterteil (20) als kinematisches Kriterium errechnet wird.

11. Verfahren nach Anspruch 10, **dadurch gekennzeichnet, dass** die Kniewinkelgeschwindigkeit und Unterteilwinkelgeschwindigkeit während des Gehens ermittelt und daraus der Quotient der beiden Winkelgeschwindigkeiten errechnet wird, wobei die Neigung des Untergrundes anhand der Änderung des Quotienten der Winkelgeschwindigkeiten ermittelt wird.

12. Verfahren nach Anspruch 8 und 10, **dadurch gekennzeichnet, dass** das Wegberechnungskriterium und das kinematische Kriterium zur Bestimmung der Untergrundneigung verwendet werden.

13. Verfahren nach einem der voranstehenden Ansprüche, **dadurch gekennzeichnet, dass** die Lage und/oder Orientierung eines Bodenreaktionskraftvektors in Bezug auf die Orthese oder Prothese als Steuergröße verwendet wird.

14. Verfahren nach Anspruch 1, **dadurch gekennzeichnet, dass** das Erkennen eines Überrollens eines Fußteils (30) über eine Kante eine Flexionswiderstandserhöhung verhindert oder den erhöhten Flexionswiderstand (Rf) wieder reduziert.

## Claims

1. A method for controlling a prosthesis or orthosis of the lower extremity, having an upper part (10) and having a lower part (20) which is connected to the upper part (20) via a knee joint (1) and is mounted so as to be pivotable relative to the upper part (10) about a joint axis (15), wherein there is arranged between the upper part (10) and the lower part (20) an adjustable resistance device (40) by means of which, during walking, a flexion resistance (Rf) is changed on the basis of sensor data in an early and mid stance phase after initial heel contact up to the mid stance phase, **characterized in that,** after the initial heel contact, the flexion resistance (Rf) is increased to a value at which further flexion is blocked or at least slowed, wherein the temporal profile of the flexion resistance increase and/or the maximum achievable flexion angle (Af) is changed in dependence on the inclination of the surface or a height difference (ΔH) to be overcome.

2. The method as claimed in claim 1, **characterized in that** the maximum achievable flexion angle (Af) and/or the flexion angle (Af) at which the maximum flexion resistance (Rf) is achieved is increased in the case of an increasingly steep surface.

3. The method as claimed in claim 1 or 2, **characterized in that**, in the case of an increasingly steep surface, the maximum flexion resistance (Rf) is reduced.

4. The method as claimed in one of the preceding claims, **characterized in that** the flexion block or the flexion resistance increase is maintained for a defined period of time, and then the flexion resistance (Rf) is reduced.

5. The method as claimed in claim 4, **characterized in that** the flexion resistance is reduced after the flexion block or after the flexion resistance increase if a measure of the transverse force in the lower part (20) exceeds a limit value dependent on the inclination of the surface and/or a leg cord (70) exceeds a forward inclination dependent on the inclination of the surface and/or a measure of the hip moment initially exceeds and then falls below a limit value.

6. The method as claimed in claim 5, **characterized in that** the measure of the transverse force is determined by means of a transverse force sensor or from a difference in transverse force components of an ankle moment and knee moment.

7. The method as claimed in one of the preceding claims, **characterized in that** the flexion resistance (Rf) is reduced after an increase to below a blocking level if a predefined flexion angle (Af) is exceeded.

8. The method as claimed in one of the preceding claims, **characterized in that** the inclination of the surface can be calculated from a vertical and/or horizontal distance travelled in the preceding swing phase by the knee joint (1), in particular by a reference point in the vicinity of the sole of the foot, or from the ratio of a vertical and horizontal distance travelled in the preceding swing phase by the knee joint (1), in particular by a reference point in the vicinity of the sole of the foot, as a displacement calculation criterion.

9. The method as claimed in claim 8, **characterized in that** the beginning of the stance phase to be controlled is determined on the basis of an axial force impulse, a plantar flexion acceleration and/or an ankle moment.

10. The method as claimed in one of claims 1 to 7, **characterized in that** the inclination of the surface is calculated from an evaluation of a flexion angle (Af) and of an absolute angle of the upper part (10) or of the lower part (20) or from the evaluation of two absolute angles of the upper part (10) and lower part (20), as a kinematic criterion.

11. The method as claimed in claim 10, **characterized in that** the knee angular velocity and lower part angular velocity during walking are determined, and the quotient of the two angular velocities is calculated therefrom, wherein the inclination of the surface is determined on the basis of the change of the quotient of the angular velocities.

12. The method as claimed in claim 8 and 10, **characterized in that** the displacement calculation criterion and the kinematic criterion are used for determining the surface inclination.

13. The method as claimed in one of the preceding claims, **characterized in that** the position and/or orientation of a ground reaction force vector in relation to the orthosis or prosthesis is used as a control parameter.

14. The method as claimed in claim 1, **characterized in that** the detection of a roll-over of a foot part (30) over an edge prevents a flexion resistance increase or reduces the increased flexion resistance (Rf) again.

## Revendications

1. Procédé de commande d'une prothèse ou d'une orthèse du membre inférieur, comprenant une partie supérieure (10) et une partie inférieure (20) reliée à la partie supérieure (10) par l'intermédiaire d'une articulation de genou (1) et montée de manière à pouvoir basculer autour d'un axe d'articulation (15) par rapport à la partie supérieure (10), un dispositif de résistance réglable (40) étant disposé entre la partie supérieure (10) et la partie inférieure (20), par l'intermédiaire duquel une résistance à la flexion (Rf) est modifiée lors de la marche sur la base de données de capteurs pendant la phase de double appui de réception et la phase de simple appui après un contact initial avec le talon jusqu'à la phase de simple appui, **caractérisé en ce qu'**après le contact initial avec le talon, la résistance à la flexion (Rf) est augmentée jusqu'à une valeur à laquelle une flexion supplémentaire est bloquée ou au moins ralentie, l'évolution dans le temps de l'augmentation de la résistance à la flexion et/ou l'angle de flexion maximal atteignable (Af) étant modifié en fonction de la pente du sol ou d'une différence de hauteur (ΔH) à surmonter.

2. Procédé selon la revendication 1,
**caractérisé en ce que** l'angle de flexion maximal atteignable (Af) et/ou l'angle de flexion (Af) auquel la résistance maximale à la flexion (Rf) est atteinte est augmenté au fur et à mesure d'une pente croissante du sol.

3. Procédé selon la revendication 1 ou 2,
**caractérisé en ce que** la résistance maximale à la flexion (Rf) est réduite au fur et à mesure d'une pente croissante du sol.

4. Procédé selon l'une des revendications précédentes,
**caractérisé en ce que** le blocage de la flexion ou l'augmentation de la résistance à la flexion est maintenu pendant une période définie, puis la résistance à la flexion (Rf) est réduite.

5. Procédé selon la revendication 4,
**caractérisé en ce que** la résistance à la flexion est réduite après le blocage de la flexion ou après l'augmentation de la résistance à la flexion lorsqu'une mesure de la force transversale dans la partie inférieure (20) dépasse vers le haut une valeur limite dépendant de la pente du sol et/ou lorsqu'un tendon de jambe (70) dépasse vers le haut une inclinaison vers l'avant dépendant de la pente du sol et/ou lorsqu'une mesure du couple de hanche dépasse une valeur limite d'abord vers le haut, puis vers le bas.

6. Procédé selon la revendication 5,
**caractérisé en ce que** la mesure de la force transversale est déterminée par un capteur de force transversale ou à partir d'une différence entre les composantes de force transversale d'un couple de cheville et d'un couple de genou.

7. Procédé selon l'une des revendications précédentes,
**caractérisé en ce que**, après une augmentation, la résistance à la flexion (Rf) est réduite jusqu'en dessous d'un niveau de blocage, lorsqu'un angle de flexion (Af) prédéfini est dépassé vers le haut.

8. Procédé selon l'une des revendications précédentes,
**caractérisé en ce que** la pente du sol est calculée, en tant que critère de calcul de la course, à partir d'une distance verticale et/ou horizontale de l'articulation du genou (1), en particulier d'un point de référence à proximité de la plante du pied, parcourue lors de la phase oscillante précédente, ou à partir du rapport entre une distance verticale et une distance horizontale de l'articulation du genou (1), en particulier d'un point de référence à proximité de la plante du pied, parcourues lors de la phase oscillante précédente.

9. Procédé selon la revendication 8,
**caractérisé en ce que** le début de la phase d'appui à commander est déterminé à l'aide d'une impulsion de force axiale, d'une accélération de la flexion plantaire et/ou d'un moment de cheville.

10. Procédé selon l'une des revendications 1 à 7,
**caractérisé en ce que** la pente du sol est calculée, en tant que critère cinématique, à partir d'une évaluation d'un angle de flexion (Af) et d'un angle absolu de la partie supérieure (10) ou de la partie inférieure (20) ou à partir de l'évaluation de deux angles absolus de la partie supérieure (10) et de la partie inférieure (20).

11. Procédé selon la revendication 10,
**caractérisé en ce que** la vitesse angulaire du genou et la vitesse angulaire de la partie inférieure sont déterminées pendant la marche pour en calculer le quotient des deux vitesses angulaires, la pente du sol étant déterminée à l'aide de la modification du quotient des vitesses angulaires.

12. Procédé selon les revendications 8 et 10,
**caractérisé en ce que** le critère de calcul de la course et le critère cinématique sont utilisés pour déterminer la pente du sol.

13. Procédé selon l'une des revendications précédentes,
**caractérisé en ce que** la position et/ou l'orientation d'un vecteur de force de réaction au sol par rapport à l'orthèse ou à la prothèse est utilisée comme variable de commande.

14. Procédé selon la revendication 1,
**caractérisé en ce que** la détection du passage d'une partie du pied (30) sur un bord empêche l'augmentation de la résistance à la flexion ou réduit à nouveau la résistance à la flexion (Rf) augmentée.
